Europäisches Patentamt

European Patent Office

Office européen des brevets

(19)

(11) Publication number: **0 048 244**
**B1**

(12) **EUROPEAN PATENT SPECIFICATION**

(45) Date of publication of patent specification: **11.07.84**

(51) Int. Cl.³: **A 61 M 15/00 //B05B3/08**

(21) Application number: **81900648.7**

(22) Date of filing: **24.03.81**

(86) International application number:
**PCT/GB81/00055**

(87) International publication number:
**WO 81/02676 01.10.81 Gazette 81/23**

(54) **MEDICAL NEBULISING APPARATUS.**

(30) Priority: 25.03.80 GB 8010010
10.04.80 GB 8011801
15.04.80 GB 8012406
16.07.80 GB 8023284
02.10.80 GB 8031725
01.12.80 GB 8038830
10.01.81 GB 8100705

(43) Date of publication of application:
**31.03.82 Bulletin 82/13**

(45) Publication of the grant of the patent:
**11.07.84 Bulletin 84/28**

(84) Designated Contracting States:
**AT CH DE FR LI NL SE**

(56) References cited:
DE - B - 1 143 969
DE - B - 1 616 198
DE - C - 23 371
DE - C - 25 422
DE - C - 317 599
DE - C - 622 621
FR - A - 534 593

(73) Proprietor: MALEM, Hilal
4 Rufford Road, Sherwood
Nottingham NG5 2NR (GB)

(72) Inventor: MALEM, Hilal
4 Rufford Road, Sherwood
Nottingham NG5 2NR (GB)

(74) Representative: MacGregor, Gordon et al,
ERIC POTTER & CLARKSON 14 Oxford Street
Nottingham, NG1 5BP (GB)

(56) References cited:
FR - A - 549 747
FR - A - 1 389 559
GB - A - 179 142
GB - A - 916 196
GB - A - 1 085 092
GB - A - 1 173 380
US - A - 3 711 071
US - A - 3 812 853

Courier Press, Leamington Spa, England.

## Description

The present invention relates to medical nebulising apparatus for inhalatory use. In this specification, the term "nebulisation" is intended to include the formation of a spray from a powder as well as from a liquid.

In the medical field, the inhalation of nebulised drugs is an accepted way of treating various medical conditions such as asthma, bronchitis, cystic fibrosis and other conditions of the respiratory tract. Existing medical nebulisers are not easy to use by all patients. They are expensive, bulky and require a separate compressed air or oxygen supply, e.g. from a compressed air container or from an electric compressor device. A patient, therefore, is unlikely to have or carry his own nebuliser and has to visit a hospital for inhalation treatment.

It has been proposed to use a rotatable disc for dispersing particles in a medical nebuliser. The material is fed in an axial direction onto the disc to disperse the particles in a large chamber and an airstream is independently generated to carry particles out of the chamber. There is generation of a large proportion of large particles and the apparatus is massive.

One common way of producing a spray is the pressurised can, commonly used, for example, for such purposes as paint spraying, air fresheners and insecticide sprays. Although these cans are readily portable and are used as inhalation devices, they suffer several drawbacks. They require a pressurised carrier gas, which itself is undesirable for environmental reasons and the pressure is excessively high, with the result that the spray particles impact in the mouth and coalesce. The devices are not reusable and they need a very high degree of coordination and effort by the patient.

The use of a rotating wheel for flinging particles into a gas stream is a very old practice in some industries, for example, for comminuting metals and electrostatic powder coating. These industrial processes are described in the following U.K. patent specifications, which have been located by searching, since the invention was made.

U.K. patent No. 1,173,380 describes an apparatus for developing electrostatic charge images which involves the use of a disc electrode in an enclosed chamber which dips, while revolving, in a dye liquid. A high voltage is applied to the electrode to cause atomization of the dye liquid and the atomised particles migrate, by virtue of an applied electrical field, to a conductive support for a recording material carrying a charge image.

U.K. patent No. 1,085,092 describes an apparatus which uses a rotating toothed-disc to direct liquid particles from a reservoir into a gas stream. This apparatus essentially requires a separate supply of gas under pressure to enable the apparatus to function.

An apparatus for comminuting molten metal for the purpose of, for example, producing an oxide of that metal is described in U.K. patent No. 519,784, and a very similar apparatus is described in U.K. patent No. 574,385. The apparatus of each of these specifications requires a vessel for containing a mass of molten metal and a rotating wheel flings the particles of the molten metal into a gas stream. Specification No. 519,784, which was published forty years ago, does fleetingly, in one sentence, consider that the rotating wheel might serve to produce the gas stream, but for these industrial processes, as for present day medical nebulisers, it is accepted that a separate gas stream-producing apparatus is essential.

DE—C—23371 for example describes a nebuliser of massive construction for medical purposes. A shaft is provided with axially spaced brush bristles, the shaft being rotatable through gearing to cause the brushes to pick up liquid particles within the chamber. These particles are thrown into a separately produced gas stream produced by connection of a gas supply to the chamber. The gas stream entrains the particles and carries them to a mouthpiece.

GB—A—179142 describes a similar device for use with powder. A brush acts to throw powder into a separately produced gas current.

US—A—3711071 uses a rotating disc to produce a milling action within a chamber for producing particles. A separately formed gas stream carries the particles through apertures in the disc to an outlet.

DE—B—1616198 provides a brush to pick up particles fed to the periphery of the brush. These particles are entrained in a separately produced airstream. In this case, the airstream is produced by a fan attached to the same shaft as the brush but in a separate chamber. There is communication between the chambers and an air inlet whose size is variable by a shutter.

The present invention provides medical nebulising apparatus comprising a housing including a chamber provided with a reservoir for material to be nebulised and having an air inlet, the chamber having an outlet passage for nebulised material leading to an outlet portion of the housing, and nebulising means in the form of a driven disc whose peripheral portion passes through the reservoir to pick up material, the chamber having a generally circular peripheral surface around the peripheral surface of the disc and the axial dimension of the chamber being less than the radial dimension, characterised in that the chamber outlet passage extends from a port in said peripheral surface of the chamber, the port being positioned in the path of particles flung from the disc, and the disc produces an airflow along the outlet passage which entrains particles of the material picked up by the disc without additional means for producing the airflow.

The passage between the chamber and the outer portion preferably includes a baffle arrangement to assist in controlling the particle size at the outlet by returning larger particles to the chamber.

Apparatus according to the invention can be made sufficiently small for carrying in a pocket or handbag and can provide a fine mist with particles not exceeding 30 $\mu$ or even not exceeding 15 $\mu$.

Reference is now made to the accompanying drawings, wherein:—

Figure 1 is a perspective view of a first embodiment of a medical nebulising apparatus according to the invention;

Figure 2 is a perspective view of a broken away portion of the apparatus of Figure 1 showing a reservoir thereof;

Figure 3 is a sectional side elevation on the line 3—3 of Figure 1;

Figure 4 is a sectional view on the line 4—4 of Figure 1;

Figures 5 and 6 are sectional views similar to Figures 3 and 4 of a second embodiment; and

Figures 7 and 8 are sectional views similar to Figures 3 and 4 of a third embodiment.

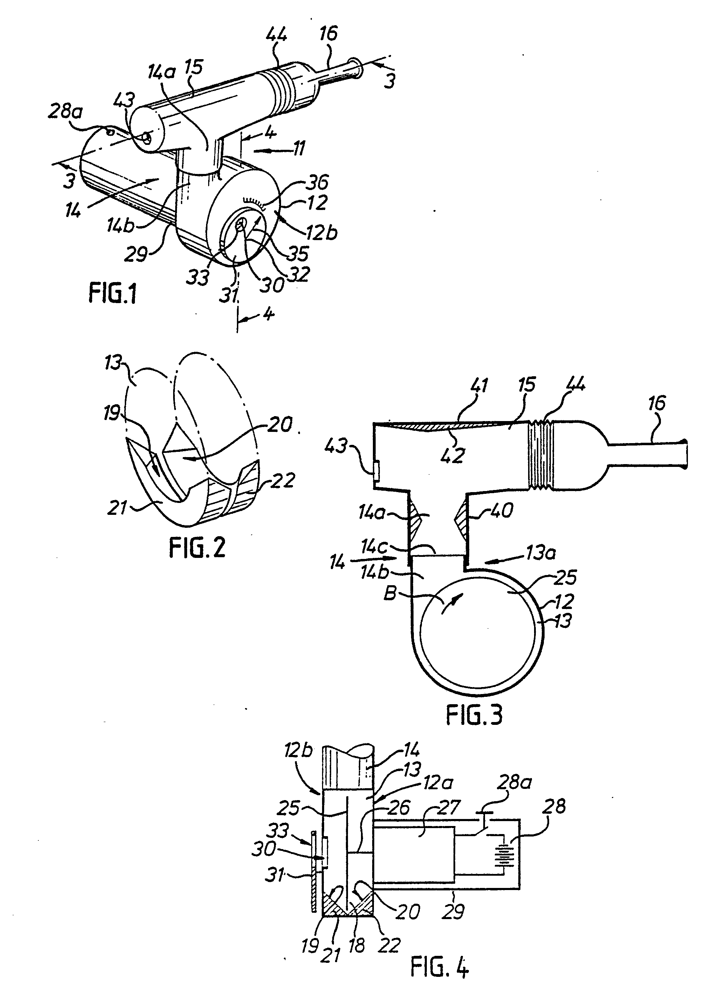

Referring to Figures 1 to 4, there is shown a medical nebulising apparatus comprising a housing 11 having a hollow cylindrical portion 12 having a chamber 13 therein. An outlet tube 14 leads upwardly (in the position of use shown in Figure 1) from the cylindrical portion to define an outlet passage tangential to the chamber 13 and communicating therewith through a port 13a. The outlet tube 14 leads to a tubular head 15, which has a T connection with the tube 14. A mouthpiece 16 is connected to one end of the head 15. In use, material to be nebulised is contained in the chamber 12 and a fine spray consisting of an air stream entraining particles of the material is provided at the mouthpiece, as described hereafter.

The lower portion of the chamber 13 opposite to the tube 14 defines a reservoir 18 and internal sloping surfaces 19, 20 cause the reservoir to narrow towards the periphery, each sloping surface being arcuate and following the internal peripheral contour of the chamber as is apparent from Figure 2. Each surface is defined in this construction by a solid internal wall 21, 22 of substantially triangular section as is apparent from Figure 4.

A nebulising disc 25 is rotatably mounted in the chamber 13 by an axle 26, so that part of the marginal periphery of the disc lies between the sloping surfaces 19, 20 in the reservoir, the diameter of the disc being only slightly less than that of the container and the general plane of the disc being parallel to the side walls 12a, 12b of the cylindrical portion 12. The axle is driven by an electrical motor 27 and a replaceable electric battery 28 mounted in a casing 29 on one side wall 12a of the cylindrical portion 12, which defines the chamber 13. A switch 28a is accessible externally of the casing and is actuable to operate the motor.

The opposite side wall 12b of this portion has a central air inlet 30, the size of which is variable by means of a shutter 31. The shutter in this embodiment is in the form of a disc pivotally mounted by pivot pin 32 on the side wall 12b. The shutter has an aperture 33 of equal diameter to the inlet 30, so that the inlet is fully open with the aperture 33 and the inlet in register. The shutter can be angularly moved to reduce the area of the inlet exposed. The shutter carries a pointer 35 associated with an arcuate scale 36 marked on the side wall 12b, so that a predetermined area of the inlet can be exposed recording the scale reading.

The outlet parts of the device have an internal baffle arrangement defined by a constriction 40 near the mouth of the outlet tube 14, adjacent the head 15, and a conical projection 41 provided in the head 15 directly opposite the outlet tube 14, the apex 42 of the projection 41 being on the axis of the outlet tube. The end of the head opposite to the mouthpiece 16 is closed except for a vent 43. A flexible bellows 44 attaches the mouthpiece 16 to the head 15.

The outlet tube 14 comprises an upper part 14a, integral with the head 15 and a lower part 14b, integral with the cylindrical portion 12. The upper and lower parts are push-fitted together. The head 15, with the upper part 14a, can, therefore, be removed, so that the mouth 14c of the lower part defines an inlet.

In use, the material to be nebulised is introduced to the reservoir 18 through the inlet mouth 14c. The patient's mouth is put to the mouthpiece 16 and the electric motor is energised by operating the switch 28a. The nebulising disc 25 is thereby rotated in the direction of arrow B in Figure 3. A part of the periphery is immersed in the material in the reservoir 18, so that the disc flings particles of the material along the tangential passage in the outlet tube 14. The rotating disc also serves as a centrifugal pump to draw air in through the inlet 30 and produce a gas stream along the passage in the outlet tube, the particles of the material being entrained in the gas stream so that nebulised material passes out of the mouthpiece 16 in a fine spray. The amount of air drawn into the chamber can be controlled by the position of the shutter 31.

As the disc flings particles of the material at a high speed, these particles (especially the larger ones) tend to impact against the inner walls of the chamber 13 and shatter into even smaller particles. The larger particles entering the tube 14 tend to coalesce and drip back into the reservoir 18 or onto the disc for further treatment. This continuous and repeated action leads to the generation of very small particles. The constriction 40 obstructs flow of larger particles and assists this action as does the conical projection 41 which serves to direct particles which coalesce there back into the reservoir. The spray at the mouthpiece may therefore be made up of particles of maximum size of 15 $\mu$.

The vent permits sucking in of extra air by a patient and also prevents a sucking action at the mouthpiece from drawing in material from the reservoir and relieves pressure, if the patient obstructs the mouthpiece or blows back into the mouthpiece. The patient can breathe freely, therefore, without interfering with the operation of the apparatus. Venting in this case also prevents wetting of the material in the reservoir from the user's breath, which is important, if the material is a powder.

The shaping of the reservoir 18 minimises changes in the depth of material therein, with changes in orientation of the apparatus.

The disc may be rotationally mounted off-centre or may be unbalanced about the axis by other means such as weighting to cause vibrations, which assist in breaking up and fluidising the material in the reservoir to improve nebulisation. This may be especially useful where the material is a powder. Large particles incapable of being nebulised in the apparatus could be contained in the reservoir to cause such vibration and assist in breaking the powder particles into even smaller ones.

These vibrations could cause unpleasant sensations in the lips of the user and the flexible bellows 44 serves to damp the vibrations at the mouthpiece.

The disc is preferably rotated at 8000 r.p.m. or more to provide a good rate of delivery of nebulised material.

The described nebulising apparatus is small and is capable of being held in a pocket or handbag. This is assisted by the connection between the tube parts 14a, 14b, which permits swivelling of the parts to make the apparatus more compact.

The motor casing 29 may be removable to facilitate sterilisation, unless the casing is watertight. The casing may have, for example, a bayonet or screw fitting with the cylindrical portion 12 and the shaft 26 may have two parts with a spline connection. The battery may be rechargeable from the mains.

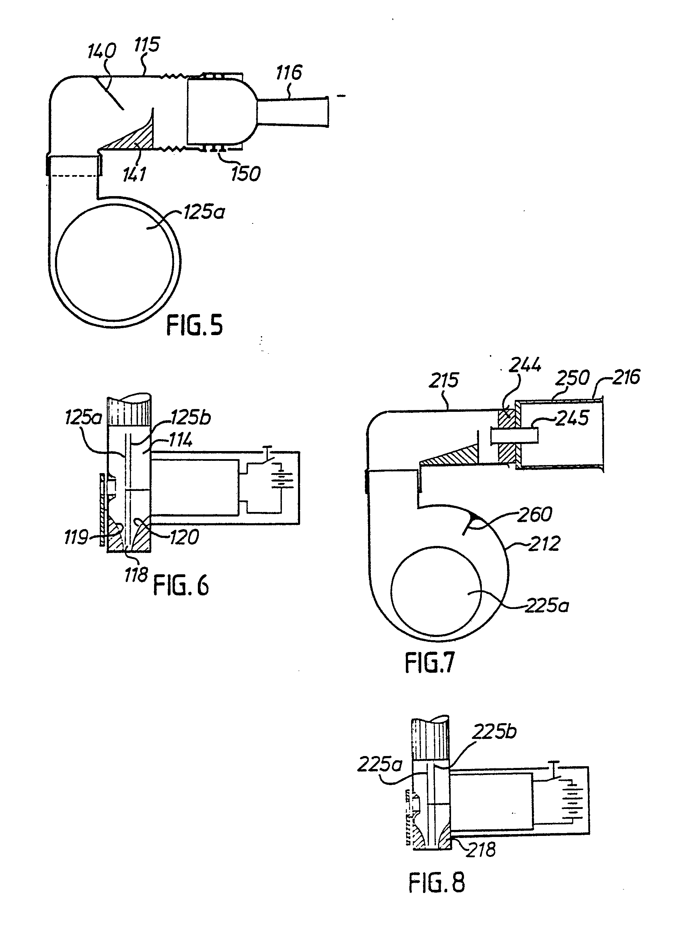

An alternative embodiment is shown in Figures 5 and 6, in which the overall construction is substantially the same as the embodiment described above. In this case there are two parallel closely spaced nebulising discs and the internal walls of the reservoir 118 are further spaced at the periphery of the chamber 114 to accommodate the two discs. In this case the walls 119, 120 are convexly curved in section instead of straight.

Instead of the projection 41 and constriction 40, baffles 140, 141 are provided in the head 115. Ventilation holes 150 are optionally provided in this case adjacent to the mouthpiece 116, the size being adjustable by relative movement of the mouthpiece 116 and the rest of the head 115.

Figures 7 and 8 show a modification of the embodiment of Figures 5 and 6, in which the nebulising discs are eccentrically positioned relative to the peripheral wall of the chamber and are of smaller diameter than in the previously described embodiment. The discs can be rotated in either direction. The discs are positioned near the bottom of the chamber to extend into the reservoir 218, as previously. In this case, a radial baffle 260 is provided in the chamber extending towards the discs. This enhances agitation and disruption of particles within the chamber as a result of impact and also redirects larger particles onto the discs for further treatment.

The mouthpiece 216, in this case is mounted in the outlet from the head 215 by means of a resilient bung 244 to absorb vibrations. An outlet nozzle 245 extends through the bung to communicate the interior of the mouthpiece with the interior of the head. Ventilation holes 250 are provided around the mouthpiece. This arrangement is preferred and is particularly effective to permit the user to breathe freely and to prevent air from the lungs of the user reaching the reservoir.

Each of the described embodiments can be easily cleaned and sterilised.

A very simple arrangement may be effective where the head, such as 15 in Figure 1, is not provided, but the outlet tube 14 itself may define the mouthpiece. The inlet for air may be defined by the outlet tube, the latter being sufficiently wide to permit sucking of air into the chamber. In this case, a mouthpiece may be provided having an inner outlet tube surrounded by venting, as shown in Figure 7.

It is envisaged that a nebuliser may be constructed according to the invention for use in an incubator.

**Claims**

1. Medical nebulising apparatus comprising a housing (11) including a chamber (12) provided with a reservoir (18) for material to be nebulised and having an air inlet (30), the chamber having an outlet passage (14) for nebulised material leading to an outlet portion (16) of the housing, and nebulising means in the form of a driven disc (25) whose peripheral portion passes through the reservoir to pick up material, the chamber having a generally circular peripheral surface around the peripheral surface of the disc and the axial dimension of the chamber being less than the radial dimension, characterised in that the chamber outlet passage (14) extends from a port (13a) in said peripheral surface of the chamber, the port being positioned in the path of particles flung from the disc, and the disc produces an airflow along the outlet passage which entrains particles of the material picked up by the disc without additional means for producing the airflow.

2. Medical nebulising apparatus according to Claim 1, in which the width of the outlet passage (14) adjacent the chamber (12) is equal to the axial width of the chamber in the same plane.

3. Medical nebulising apparatus according to Claim 2, wherein the reservoir is defined by an axially narrowed portion (21, 22) of the chamber.

4. Medical nebulising apparatus according to Claim 1, 2 or 3 wherein the rotatable member comprises a pair of closely spaced generally parallel discs (125a, 125b) mounted on a common axis of rotation.

5. Medical nebulising apparatus according to any preceding claim, wherein the air inlet is in a wall (12b) of the chamber and its effective area is selectively variable.

6. Medical nebulising apparatus according to Claim 5, comprising a shutter (31), the wall (12b) of the chamber and the shutter being provided one with a pointer (35) and the other with a scale (36) for indicating selectable positions of the shutter.

7. Medical nebulising apparatus according to any preceding claim, wherein the rotatable element is unbalanced to produce vibrations in the reservoir and vibration damping means (44) is provided between the outlet portion (16) and the housing (11).

8. Medical nebulising apparatus according to any preceding claim, including a baffle arrangement (40, 41) to assist return of larger particles to the chamber, so as to control the size of particles of material in the air stream to a maximum of 30 $\mu$.

9. Medical nebulising apparatus according to any preceding claim, wherein the passage is defined by a first portion (14) leading from the chamber (12) and a second portion (15) generally perpendicular thereto and leading to the outlet portion (16).

10. Medical nebulising apparatus according to Claim 9 including one or more vents (43, 150) in the second portion (15) or in the outlet portion (116).

## Revendications

1. Nébuliseur médical comprenant un carter (11) contenant une chambre (12) pourvue d'un réservoir (18) de matière à nébuliser et comportant une arrivée (30) d'air, cette chambre étant équipée d'un passage (14) de sortie de la matière nébulisée conduisant à une partie (16) de sortie du carter, et un moyen de nébulisation en forme de disque tournant (25) dont la partie périphérique passe dans le réservoir pour y capter la matière, la chambre présentant une surface périphérique circulaire dans son ensemble et qui entoure la surface périphérique du disque, la dimension axiale de cette chambre étant inférieure à sa dimension radiale, ce nébuliseur étant caractérisé en ce que le passage (14) de sortie de la chambre part d'un orifice (13a) formé dans la surface périphérique de celle-ci, cet orifice étant situé sur le trajet des particules projetées par le disque; et en ce que le disque produit le long du passage de sortie un courant d'air qui entraîne les particules de matière captées par le disque sans moyen supplémentaire pour produire le courant d'air.

2. Nébuliseur médical suivant la revendication 1, dans lequel la largeur du passage (14) de sortie au voisinage de la chambre (12) est égale à la largeur axiale de la chambre dans le même plan.

3. Nébuliser médical suivant la revendication 2, dans lequel le réservoir est formé par une partie axialement rérécie (21, 22) de la chambre.

4. Nébuliseur médical suivant l'une quelconque des revendications 1, 2 ou 3, dans lequel l'élément tournant comprend deux disques généralement parallèles (125a, 125b) montés sur un axe commun de rotation.

5. Nébuliseur médical suivant l'une quelconque des revendications précédentes, dans lequel l'arrivée d'air est située dans une paroi (12b) de la chambre et sa section libre effective de passage est sélectivement variable.

6. Nébuliseur médical suivant la revendication 5, comprenant un volet (31), la paroi (12b) de la chambre et le volet comportant l'un une aiguille (35) et l'autre une graduation (36) pour indiquer les positions sélectionnées du volet.

7. Nébuliseur médical suivant l'une quelconque des revendications précédentes, dans lequel l'élément tournant est déséquilibré pour créer des vibrations dans le réservoir et un moyen (44) d'amortissement de ces vibrations est prévu entre la partie (16) de sortie et le carter (11).

8. Nébuliseur médical suivant l'une quelconque des revendications précédentes, comprenant un montage déflecteur (40, 41) pour faciliter le renvoi des particules plus grosses vers la chambre, de manière à limiter la dimension des particules de matière dans le courant d'air à un maximum de 30 $\mu$.

9. Nébuliseur médical suivant l'une quelconque des revendications précédentes, dans lequel le passage est constitué d'une première partie (14) venant de la chambre (12), et d'une deuxième partie (15) perpendiculaire dans son ensemble à la première partie et conduisant à la partie (16) de sortie.

10. Nébuliseur médical suivant la revendication 9, comprenant un ou plusieurs orifices (43, 150) de ventilation dans la deuxième partie (15) ou dans la partie (116) de sortie.

## Patentansprüche

1. Medizinisches Zerstäubergerät mit einem Gehäuse (11), einer Kammer (12) mit einem darin vorgesehenen Vorratsbehälter (18) für das zu zerstäubende Material und mit einem Lufteinlaß (30), wobei die Kammer mit einem in den Auslaßteil (16) des Gehäuses führenden Auslaßdurchtritt (14) für das zu zerstäubende Material versehen ist und Zerstäubungseinrichtungen in Form einer angetriebenen Scheibe (25) vorhanden sind, deren Rand durch den Vorratsbehälter läuft und Material aufnimmt und die Kammer um den Rand der Scheibe eine im wesentlichen kreisförmige Oberfläche bildet, wobei die axiale Ausdehnung der Kammer geringer ist als ihre radiale Ausdehnung, dadurch gekennzeichnet, daß sich der Kammerauslaßdurchtritt (14) von einem Auslaß (13a) in der besagten peripheren Oberfläche der Kammer (14) aus erstreckt und der Auslaß im Weg der von der Scheibe abfliegenden Partikel angeordnet ist und die Scheibe einen Luftstrom längs des Auslaßdurchtritts erzeugt, welcher die von der Scheibe aufgenommenen Materialpartikel erfaßt ohne das zusätzliche Einrichtungen zur Erzeugung des Luftstromes erforderlich sind.

2. Medizinisches Zerstäubergerät nach Anspruch 1, dadurch gekennzeichnet, daß die Breite des der Kammer (12) benachbarten Auslaßdurchtritts (14) gleich der axialen Breite der Kammer in der gleichen Ebene ist.

3. Medizinisches Zerstäubergerät nach Anspruch 2, dadurch gekennzeichnet, daß der Vorratsbehälter durch einen axial verengten Abschnitt (21, 22) der Kammer gebildet ist.

4. Medizinisches Zerstäubergerät nach Anspruch 1, 2 oder 3, dadurch gekennzeichnet, daß das rotierende Teil aus einem Paar nahe beieinander paralleler auf einer gemeinsamen Rotationsachse angeordneter Scheiben (125a, 125b) gebildet ist.

5. Medizinisches Zerstäubergerät nach einem der vorausgehenden Ansprüche 1 bis 4, dadurch gekennzeichnet, daß der Lufteinlaß in der Wand (12b) der Kammer vorgesehen ist, wobei dessen wirksame Fläche einstellbar ist.

6. Medizinisches Zerstäubergerät nach Anspruch 5, dadurch gekennzeichnet, daß eine Blende (31) vorgesehen ist und die Wand (12b) der Kammer der Blende jeweils mit einem Zeiger (35) bzw. mit einer Skala (36) zur Anzeige der jeweiligen Blendenstellung vorgesehen sind.

7. Medizinisches Zerstäubergerät nach einem der vorausgehenden Ansprüche 1 bis 6, dadurch gekennzeichnet, daß das rotierende Bauteil nicht ausgewuchtet ist um Vibrationen im Vorratsbehälter zu erzeugen und zwischen dem Auslaßteil (16) und dem Gehäuse (11) Schwingungsdämpfungseinrichtungen (44) vorgesehen sind.

8. Medizinisches Zerstäubergerät gemäß einem der vorausgehenden Ansprüche 1 bis 7, dadurch gekennzeichnet, daß eine Drosseleinrichtung (40, 41) vorgesehen ist, um die Rückführung größerer Partikel zur Kammer zu unterstützen, so daß die Größe der Materialpartikel im Luftstrom eine Maximalgröße von 30 $\mu$m nicht überschreitet.

9. Medizinisches Zerstäubergerät nach einem der vorausgehenden Ansprüche 1 bis 8, dadurch gekennzeichnet, daß der Durchtritt durch einen ersten Abschnitt (14), welcher zur Kammer (12) führt und einen zweiten Abschnitt (15) bildet, welcher im wesentlichen rechtwinklig hierzu verläuft und zum Auslaßabschnitt (16) führt.

10. Medizinisches Zerstäubergerät nach Anspruch 9, dadurch gekennzeichnet, daß im zweiten Abschnitt (15) oder im Auslaßabschnitt (116) einen oder mehrere Öffnungen (43, 150) vorgesehen sind.

FIG.1

FIG.2

FIG.3

FIG.4

FIG.5

FIG.6

FIG.7

FIG.8